# EUROPEAN PATENT APPLICATION

(11) **EP 4 056 171 A1**
(43) Date of publication of application: **14.09.2022**
(21) Application number: 22156165.7
(22) Date of filing: 10.02.2022
(51) Int. Cl.: A61K 9/20, A61K 31/216

(54) **TASTE MASKED RACECADOTRIL TABLET COMPOSITION AND PROCESS FOR PREPARATION THEREOF**

(30) Priority: 12.03.2021 IN 202111010560
(71) Applicant: Athena Pharmaceutiques SAS, 78430 Louveciennes (FR)
(72) Inventor: Chaudhari, Mahendra B., 400602 Thane (West) Maharashtra (IN); Shahane, Anita Kunal, 421301 Kalyan (West) Maharashtra (IN)
(74) Representative: Dr. Solf & Zapf Patent- und Rechtsanwalts PartG mbB

(57) **Abstract**

The present invention relates to a taste masked Racecadotril tablet composition comprising: 13 to 15 % w/w of Racecadotril; 0.3 to 0.4 % w/w of polyvinylpyrrolidone; 3 to 5 % w/w of anhydrous colloidal silica; 50 to 60 % w/w of mannitol; 0.5 to 1% w/w of polyethylene glycol; 4 to 5 % amino alkyl methacrylate copolymer; 5 to 10 % w/w of microcrystalline cellulose; and 3 to 7 % w/w of croscarmellose cellulose; wherein the % w/w is with respect to the total weight of the composition.

## Description

### FIELD OF THE INVENTION

The present invention relates to a taste masked Racecadotril tablet composition and process for preparation thereof. The invention also relates to tablet of Racecadotril in the form of an orally disintegrating tablet or dispersible tablet and a process of preparation thereof.

### BACKGROUND OF THE INVENTION

Racecadotril (disclosed in EP038758BI) is an antidiarrheal drug which acts as an enkephalinase inhibitor which acts by reducing the secretion of water and electrolytes into the intestine. Chemically it is (RS)-Benzyl N-[3-(acetylthio)-2-benzylpropanoyl]glycinate with following chemical structure.

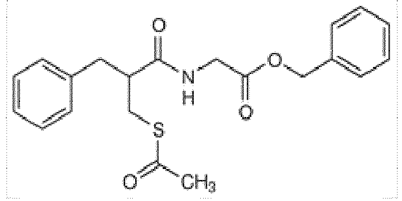

It is available commercially as Hidrasec^{®} and Tiorfan^{®} in sachet dosage forms. Racecadotril being a hydrophobic substance having bitter obnoxious taste represents difficulties in preparing a suspension dosage form. Hence, it is formulated as a sachet and conventional tablet dosage form.

Racecadotril also called as acetorphan is an oral enkephalinase inhibitor for use in the treatment of acute diarrhea. By preventing the degradation of endogenous enkephalins, Racecadotril reduces hypersecretion of water and electrolytes into the intestinal lumen.

Orally disintegrating tablets (ODT), differ from conventional tablets in that they disintegrate upon contact with mouth saliva.

Dispersible tablets (DT), differ from conventional tablets in that they are designed to be dissolved/disintegrated in a liquid. Accordingly, orally dispersible tablets have been developed such that the same medicine can be orally administered in a simple and effective manner to children, adults and people with impaired swallowing function and similar conditions. Even a patient who is not suffering from any swallowing limitation will find it much easier to take it as oral disintegrating or dispersible tablet compared to a conventional tablet.

Racecadotril has an unpleasant strong bitter taste and after taste. This increases the complexity and difficulty in preparation of a suitable oral pharmaceutical formulation of Racecadotril. Various taste masking techniques are available like polymer coating, hot-melt extrusion, microencapsulation, complexation, and spray-drying. However, these processes are complex and also increase the cost and time for the product development and manufacturing. It is therefore highly desirable to develop a Racecadotril composition which has taste masking properties while permitting rapid release of the drug from the granules and allowing rapid absorption in the body after oral administration.

EP2749270 discloses a dispersible tablet comprising Racecadotril whose taste is masked wherein the taste is masked by coating with an acrylic acid polymer or a cellulose polymer by wet granulation method without being mixed with any excipient. Though it reduces the bitter taste being a conventional granulation technique it is not efficient in covering all the drug particles by coating material and thus results in perceptible bitter taste.

EP1294372 discloses dry powder formulation of Racecadotril wherein the characteristic of the formulation is that, the Racecadotril granulate prepared with a suitable carrier is coated and mixed with a sweetener. Acrylate and methacrylate polymers especially Eudragit NE 30D are stated as coating agents. According to the procedure, first, Racecadotril granulate is prepared with a little sugar, it is coated with a coating agent; the coated granule is mixed with the remaining sugar, Aerosil and sweetener and then filled into sachet. This technique has difficulty to provide high dose drug required for young children and adults and when higher dose is prepared have same drawback of the formulation described in EP2749270.

Indian patent application 1652/MUM/2006 discloses a taste-masked rapidly disintegrating pharmaceutical composition of Racecadotril; process for preparing such composition and a method of using such composition.

Chinese patent document CN1506043A discloses pharmaceutical preparation, relating to a quick disintegrating tablet containing active ingredient Racecadotril, wherein the tablet disintegrates in less than 1 minute time in contact with the saliva in the oral cavity.

Racecadotril is available in the market in the form of capsules, tablets, dry powder for suspension. Of these dosage forms tablets and capsules are not regarded as safe for younger children and also pose difficulty for elderly patients and for those who have difficulty to swallow. Though, Racecadotril is available in the form of oral powder for suspension in the form of 10 to 30 mg dose, due to its lower drug content it is unsuitable for elderly patients and younger children who usually require a dose between 100 to 175 mg which is available only in capsule and tablet dosage form.

Accordingly, there is a need for a new dosage form that will be commercially available for oral administration with good patient convenience and acceptance especially for elderly patients and younger children.

Another difficulty in the formulation of Racecadotril in oral pharmaceutical formulation is its unpleasant strong bitter taste and after taste. It is therefore highly desirable to develop coated granules of Racecadotril which have taste masking properties while permitting rapid release of the drug from the granules and allowing rapid absorption in the body after oral administration, similar to its capsule and tablet dosage form.

Accordingly, there is need for a taste masked Racecadotril tablet composition which is palatable, tastes better, disintegrates fast, has good flowability and mixing properties and stability of Racecadotril active in the formulation.

### SUMMARY OF THE INVENTION

To achieve the foregoing and other objects and needs, the present invention provides a taste masked Racecadotril tablet composition comprising Racecadotril granules with a taste masking coating and a process of preparation thereof.

In an embodiment, the present invention relates to a taste masked Racecadotril tablet composition comprising: 13 to 15 % w/w of Racecadotril; 0.3 to 0.4 % w/w of polyvinylpyrrolidone; 3 to 5 % w/w of anhydrous colloidal silica; 50 to 60 % w/w of mannitol; 0.5 to 1 % w/w of polyethylene glycol; 4 to 5 % w/w amino alkyl methacrylate copolymer; 5 to 10 % w/w of microcrystalline cellulose; and 3 to 7 % of croscarmellose sodium, wherein the % w/w is with respect to the total weight of the composition.

In another embodiment, the present invention relates to a process for preparing taste masked Racecadotril tablet, the process comprising: preparing Racecadotril granules by mixing it with mannitol and wet granulation of racecadotril, mannitol, anhydrous colloidal silica and polyvinylpyrrolidone; applying a taste masking coating suspension consisting of an amino alkyl methacrylate copolymer, polyethylene glycol and anhydrous colloidal silica on the Racecadotril granules to obtain taste masked Racecadotril granules; preparing a flavored blend by mixing mannitol, microcrystalline cellulose, croscarmellose sodium, sweetener, anhydrous colloidal silica and flavorant; blending the taste masked Racecadotril granules with the flavored blend to obtain an intermediate blend of taste masked Racecadotril granules; lubricating the intermediate blend of taste masked Racecadotril granules with magnesium stearate to form lubricated taste masked Racecadotril granules; and compressing the lubricated taste masked granules to form taste masked Racecadotril tablet.

### BRIEF DESCRIPTION OF THE DRAWINGS

The advantages and features of the present invention will become better understood with reference to the following detailed description and claims taken in conjunction with the accompanying drawing, in which:
FIG. 1 illustrates a process flow for preparation of a taste masked Racecadotril tablet composition, in accordance with an exemplary embodiment of the present invention;
FIG. 2 illustrates details for preparation of Racecadotril granules of process of FIG. 1;
FIG. 3 illustrates details of preparation of taste masked Racecadotril granules of process of FIG. 1;
FIG. 4 illustrates details of preparation of a flavored blend of process of FIG. 1;
FIG. 5 illustrates details of preparation of an intermediate blend of taste masked Racecadotril granules of process of FIG. 1;
FIG. 6 illustrates details of lubrication of the intermediate blend of taste masked Racecadotril granules of process of FIG. 1;
FIG. 7A illustrates details of compressing the lubricated taste masked Racecadotril granules of process of FIG. 1 for 175 mg; and
FIG. 7B illustrates details of compressing the lubricated taste masked Racecadotril granules of process of FIG. 1 for 100 mg.

### DETAILED DESCRIPTION OF THE INVENTION

The exemplary embodiments described herein detail for illustrative purposes are subject to many variations in structure and design. It should be emphasized, however, that the present invention is not limited to taste masked composition comprising Racecadotril, as described. It is understood that various omissions and substitutions of equivalents are contemplated as circumstances may suggest or render expedient, but these are intended to cover the application or implementation without departing from the spirit or scope of the claims of the present invention. Also, it is to be understood that the phraseology and terminology used herein is for the purpose of description and should not be regarded as limiting.

The use of terms "including," "comprising," or "having" and variations thereof herein is meant to encompass the items listed thereafter and equivalents thereof as well as additional items. Further, the terms, "a" and "an" herein do not denote a limitation of quantity, but rather denote the presence of at least one of the referenced items.

Further, the terms, "a" and "an" herein do not denote a limitation of quantity, but rather denote the presence of at least one of the referenced items.

As used herein, "about" will be understood by persons of ordinary skill in the art and will vary to some extent depending upon the context in which it is used. If there are uses of the term in which are not clear to persons of ordinary skill in the art, given the context in which it is used, "about" will mean up to plus or minus 10% of the particular term.

The present invention provides a taste masked Racecadotril tablet composition which is palatable, tastes better, disintegrates fast, has good flowability and mixing properties and stability of Racecadotril active in the formulation. The Applicant has found that these characteristics can be obtained by formulating a tablet that contains Racecadotril in the form of taste masked coated granules and a mixture of excipients.

The present invention relates to a taste masked Racecadotril tablet composition comprising: Racecadotril granules with a taste masking coating; at least one binder; and optionally a diluent and an antistatic agent.

In an embodiment, the taste masked Racecadotril tablet composition comprises: 13 to 15 % w/w of Racecadotril; 0.3 to 0.4 % w/w of polyvinylpyrrolidone; 3 to 5 % w/w of anhydrous colloidal silica; 50 to 60 % w/w of mannitol; 0.5 to 1 % w/w of polyethylene glycol; 4 to 5 % w/w amino alkyl methacrylate copolymer; 5 to 10 % w/w of microcrystalline cellulose; 3 to 7 % w/w of croscarmellose sodium; wherein the % w/w is with respect to the total weight of the composition.

As used herein, Racecadotril includes free base as well as its pharmaceutically acceptable salts, enantiomers, polymorphic forms.

Polyvinylpyrrolidone (PVP) is used as a binder. Binders may include, but are not limited to, polyvinylpyrrolidone (PVP), methylcellulose, hydroxypropyl methyl cellulose, hydroxyl propyl cellulose, sugar and combination thereof. Binders are commonly used in wet granulation to create a more effective and predictable granule formation. Binder acts as an adhesive to literally "bind together" granules and other dry ingredients to impart to the product the necessary mechanical strength. In a specific example, the binder is PVPK-30.

Mannitol is used as a diluent. Diluents may include, but are not limited to, lactose, sucrose and polyols with particle size less than 100 µm. The polyol is selected from the group consisting of mannitol, xylitol, sorbitol, maltitol, and combination thereof. A diluent enhances the granulation property of Racecadotril. In a specific example, the diluent is Mannitol Fine 35. In another specific example, the diluent is mannitol granular (PEARLITOL^{®} SD 200).

Anhydrous colloidal silica is used as an antistatic agent. The antistatic agents include, but are not limited to, micronized and non- micronized talc, fumed silica (Aerosil R 972), colloidal silica (Aerosil 200), precipitated silica (Syloid FP 244), and combination thereof. Antistatic agent is present in the formulation in the range of 0.5 to 5.0 % of the final tablet weight. Antistatic agent is incorporated in the granules to improve the flow of the material. In a specific example, the antistatic agent is Aerosil 200.

Further, in the composition, the taste masking coating consists of: amino alkyl methacrylate copolymer as a water insoluble film forming agent; polyethylene glycol as a water-soluble pore forming agent; and anhydrous colloidal silica as an antistatic agent.

Amino alkyl methacrylate copolymer is used as a film forming agent. Film forming agent include, but are not limited to, acrylic polymers, cellulosic polymers and their combination thereof. In a specific example, the film forming agent is Eudragit E 100. In alternate embodiments, among the acrylic polymers, insoluble acrylates ammonio -methacryllate copolymers (Eudragit RL 100 or RS 100 or Eudragit RS 30 D or Eudragit RL 30 D) may be used.

In a specific example, the pore forming agent is polyethylene glycol 6000 (PEG 6000).

In alternate embodiments, the taste masking coating may further comprise a permeablising agent, plasticizers, soluble agent and a surfactant. The plasticizer is selected from the group consisting of triacetin, triethycitrate, ethylphthalate, macrogols, and combination thereof. The plasticizer is used in proportion of at most 30 %, preferably 20 % of the weight of coating polymer. The soluble agents are selected in particular among the polyols having carbon atoms less than 13. The surfactants may be anionic, cationic, non-ionic or amphoteric in nature.

Croscarmellose sodium is used as disintegrating agent. The disintegrants include, but are not limited to, starch, croscarmellose sodium, polyvinyl pyrrolidone, sodium starch glycolate and other materials known to one ordinarily skilled in the art and combinations thereof. Croscarmellose is available as e.g. Ac-di-Sol or Vivasol GF, Crospovidone is available as Kollidon CL. The proportion of the disintegrating agent ranges from 3 to 15 %, more preferably from 3 to 10 %. In case of combinations, each disintegrating agent comprises between 1% to 10% of the weight of the tablet. In a specific example, the disintegrating agent is VIVASOL^{®} GF.

Croscarmellose sodium is also considered as "super disintegrant" as it is very effective even at low concentrations and it promotes the tablet to breakdown. It is manufactured from cellulose (plant fibre) and is derived from internally crosslinking cellulose ether, sodium carboxymethylcellulose, which is a water-soluble polymer. Due to Cross-linking croscarmellose sodium exhibits excellent swelling properties. It is effective when used in intra-granular or extra-granular portion of the formulation. CCS is the most effective product available because of the strong swelling force. Croscarmellose sodium is a very effective super disintegrant as it lets water penetration into tablets through the hydrophilic, fibrous particles and the subsequent development of strong disintegration force. The commercially available grades of croscarmellose sodium can be used.

The soluble diluent agent which also forms the body of the tablet matrix consist of polyols having less than 13 carbon atoms and being in the form of directly compressible product having average particle size of 100 µm to 500 µm. The polyol is selected from the group comprising mannitol, xylitol, sorbitol, maltitol, and combination thereof. The more preferable soluble diluent is mannitol. The ratio of the soluble diluent in the tablet maybe from 20 % to 80 %, more preferably from 50% to 60%.

Microcrystalline cellulose is used as a cushioning agent. Microcrystalline cellulose is a pure partially depolymerized cellulose synthesized from α-cellulose precursor (type Iβ), obtained as a pulp from fibrous plant material, with mineral acids using hydrochloric acid to reduce the degree of polymerization. Beyond an anti-adherent, microcrystalline cellulose is most widely used for direct compression and also serves as a strong dry binder, tablet disintegrant, absorbent, filler or diluent. The commercially available grades of microcrystalline cellulose can be used. In a specific example, the cushioning agent is MCC 102 (Vivapur pH102).

Cushioning agent which also acts as swelling agent incorporated in the mixture of excipients to prevent the damage to the taste mask coating of the Racecadotril granules during its compression in the tablets. Microcrystalline cellulose (MCC) is the material used for this purpose in the range of 2 to 10 % of the tablet weight, more preferable from 5 to 10 %.

The sweetening agents (also known as sweeteners) include, but are not limited to, aspartame, potassium acesulfame, sodium saccharine, sucralose, neohisperidin, monoammonium glycyrrhizinate, and other materials known to one ordinarily skilled in the art and combinations thereof. Sweetener is present in the formulation from 0.5 to 5 %, more preferable 4 %.

Lubricants may be selected from, but are not limited to, those conventionally known in the art such as Mg (magnesium), Al (aluminum) or Ca (calcium) or Zn (zinc) stearate, sodium stearyl fumarate, stearic acid, sodium benzoate, micronized macrogols, and other materials known to one ordinarily skilled in the art and combinations thereof. The amount of lubricant is from 0 to 3 %, more preferably from 0 to 2 % and preferably 1 %.

The flavorants include natural and artificial flavors. These flavors include, but are not limited to synthetic flavor oils and flavoring aromatics, and/or oils, oleo resins and extracts derived from plants, leaves, flowers, fruits, etc., and other materials known to one ordinarily skilled in the art and mixtures thereof. Representative flavor oils include: spearmint oil, cinnamon oil, peppermint oil, clove oil, bay oil, thyme oil, cedar leaf oil, oil of nutmeg, oil of sage, and oil of bitter almonds. Also useful are artificial, natural or synthetic fruit flavors such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavorings can be used individually or in admixture. Commonly used flavors also include mints such as peppermint, artificial vanilla, cinnamon derivatives, and various fruit flavors, whether employed individually or in admixture. Flavorings such as aldehydes and esters including cinnamyl acetate, cinnamaldehyde, citral, diethylacetal, dihydrocarvyl acetate, eugenyl formate, p-methylanisole, and so forth may also be used.

In another aspect of the embodiment, the coated Racecadotril granules have particle size distribution between 100 µm to 500 µm and preferably at least 70 % of the granules have particle size ranging from 150 µm to 425 µm. The particle size of coated granules of Racecadotril is adapted to get effective taste masking with an acceptable coating percentage resulting in good mouthfeel.

In an aspect of the embodiment, the taste masked tablet disintegrates in the buccal cavity in less than 60 seconds upon contact with saliva and forms an easy to swallow pasty mass.

In another aspect of the embodiment, the disintegration time for the tablet is less than 3 minutes.

In an aspect of the embodiment, the disintegration time for the tablet is less 60 second.

In another aspect of the embodiment, the hardness of the tablet is between about 40 Newtons to about 100 Newtons.

In another aspect of the embodiment, the amount of Racecadotril is 100 mg to 200 mg per tablet.

In another embodiment, the present invention provides a process for preparing taste masked Racecadotril tablet. The process comprises: preparing Racecadotril granules by mixing and wet granulation of Racecadotril, mannitol, anhydrous colloidal silica and polyvinylpyrrolidone; applying a taste masking coating suspension consisting of an amino alkyl methacrylate copolymer, polyethylene glycol and anhydrous colloidal silica on the Racecadotril granules to obtain taste masked Racecadotril granules; preparing a flavored blend by mixing mannitol, microcrystalline cellulose, croscarmellose sodium, sweetener, anhydrous colloidal silica and flavorant; blending the taste masked Racecadotril granules with the flavored blend to obtain an intermediate blend of taste masked Racecadotril granules; lubricating the intermediate blend of taste masked Racecadotril granules with magnesium stearate to form lubricated taste masked Racecadotril granules; and compressing the lubricated taste masked granules to form taste masked Racecadotril tablet.

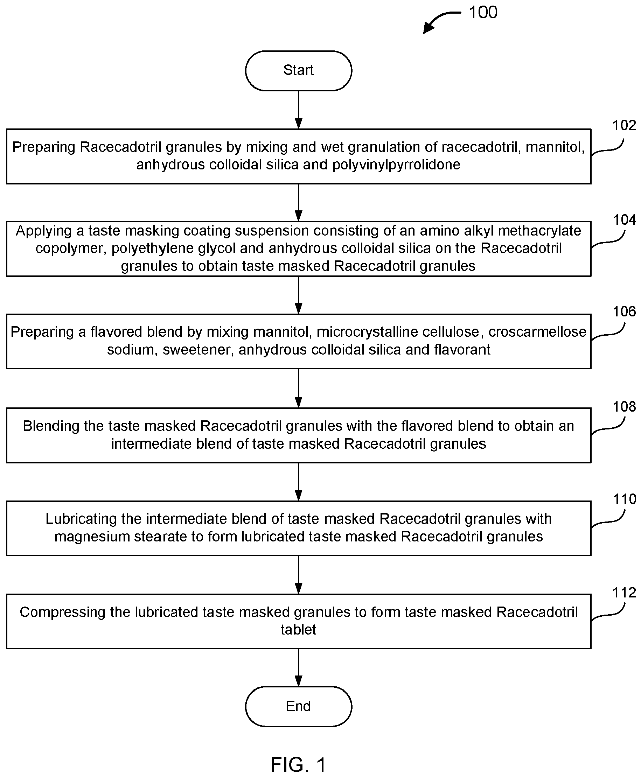

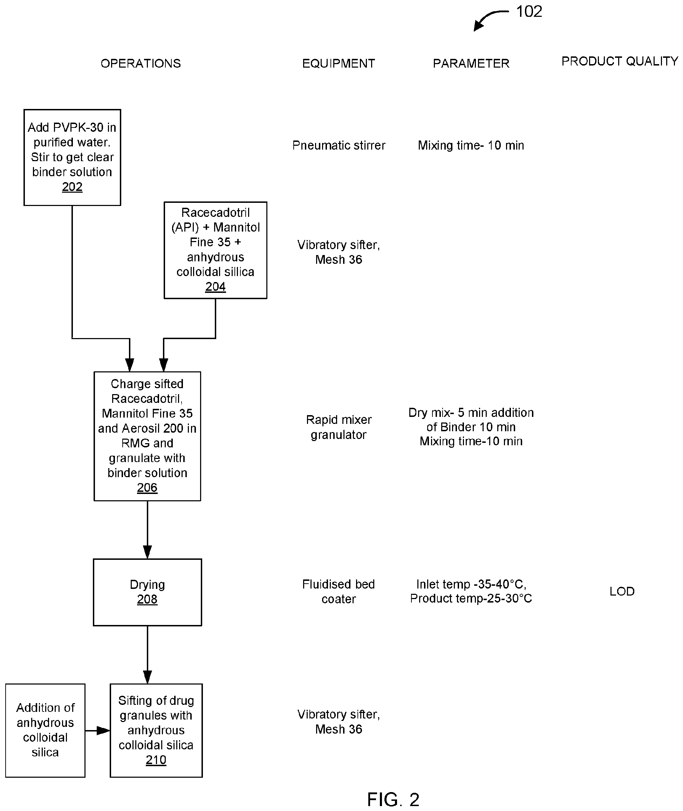

Referring to FIG. 1, at step 102, the process 100 initiates by preparing Racecadotril granules by mixing and wet granulation. The step 102 is described in detail in FIG. 2. Referring to FIG. 2, at step 202, a binder solution is prepared by adding polyvinylpyrrolidone (for example, PVPK-30) in purified water and stirring for 10 minutes to get a clear solution. Next at step 204, a process of sifting is carried out in a mixture of Racecadotril, mannitol (for example, Mannitol Fine 35) and anhydrous colloidal silica (for example, Aerosil 200).

At step 206, the granulation is carried out in a rapid mixer granulator by mixing the binder solution from step 202 with the mixture of step 204. Next, at step 208, drying is carried out in a fluidized bed drier. As used herein, in a non-limiting example, pre-determined parameters include an inlet temperature of about 35°C to about 40°C and product temperature of about 25°C to about 30°C. Next, at step 210, anhydrous colloidal silica (for example, Aerosil 200) is added to dried drug granules and is sifted to prepare the Racecadotril granules.

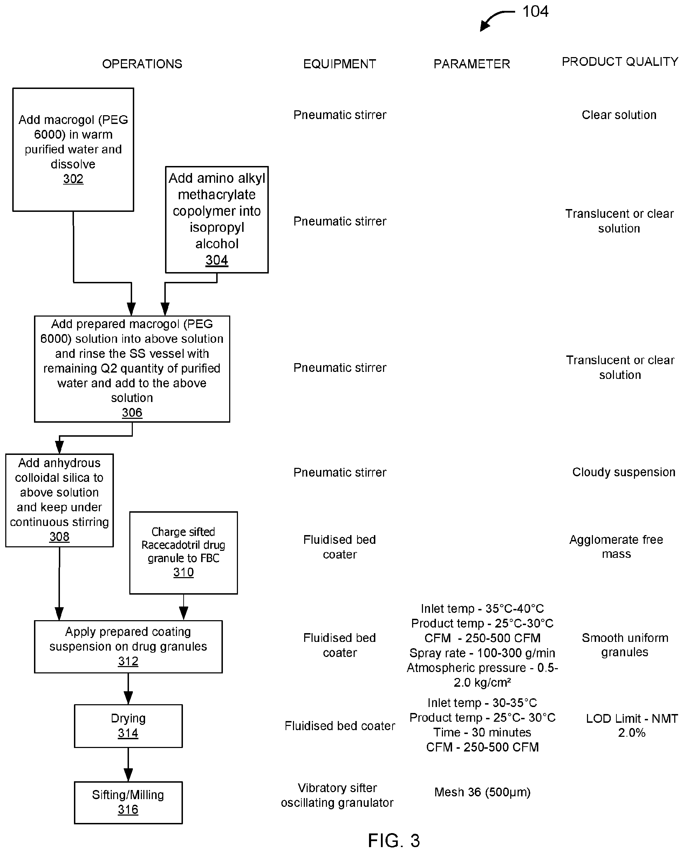

Again, referring to FIG. 1, at step 104, the next step in the process comprises applying taste masked coating suspension on the Racecadotril granules to obtain taste masked Racecadotril granules. The step 104 is described in detail in FIG. 3. Referring to FIG. 3, at step 302, a polyethylene glycol (for example, PEG 6000) is added in warm purified water and dissolved using stirring process to get polyethylene glycol solution. At step 304, amino alkyl methacrylate copolymer (for example, Eudragit E 100) is added into isopropyl alcohol and stirred to get a copolymer solution. Both the solution from step 302 and step 304 are mixed at step 306 by continuous stirring process. Further, at step 308, anhydrous colloidal silica is added to the solution of the step 306 to obtain a taste masking coating suspension. At step 310, the Racecadotril granules are processed in a fluidized bed coater.

Next, at step 312, the taste masking coating suspension is applied on Racecadotril granules in fluidized bed coater at pre-determined parameters to form coated granules. As used herein, in a non-limiting example, pre-determined parameters include an inlet temperature of about 35°C to about 40°C, product temperature of about 25°C to about 30°C, fluidization of air volume of about 250-500 CFM, spraying rate of the prepared coating suspension of about 100 to about 300 g/min and atmospheric pressure of about 0.5 to about 2.0 kg/cm² to get smooth uniform granules. Although particular pre-determined parameters are mentioned above, it will be evident to a person skilled in the art to modify predetermined parameters based on obvious variations.

Next, at step 314, the coated granules are dried in the fluidized bed coater at the pre-determined parameters. As used herein, in a non-limiting example, pre-determined parameters include an inlet temperature of about 30°C to about 35°C, product temperature of about 25°C to about 30°C, fluidization of air volume of about 250-500 CFM. Although particular pre-determined parameters are mentioned above, it will be evident to a person skilled in the art to modify predetermined parameters based on obvious variations. Finally, at step 316, the coated granules is sifted in a vibratory sifter to obtain taste masked Racecadotril granules.

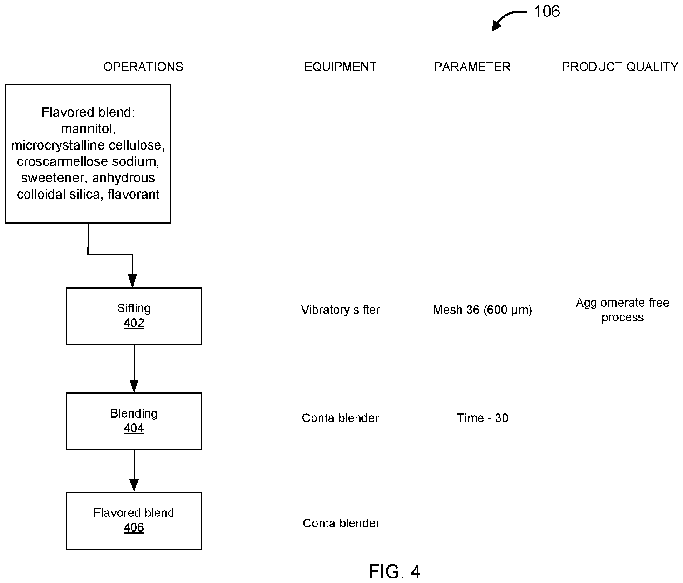

Now referring again to FIG. 1, at step 106, the process 100 comprises preparing a flavored blend. The Step 106 is described in detail in FIG. 4. At step 402, a mixture of mannitol (for example, mannitol granular), microcrystalline cellulose (for example, Vivapur PH102 or Avicel PH102), croscarmellose sodium (for example, VIVASOL^{®} GF), sweetener (for example, a combination of sucralose and aspartame), anhydrous colloidal silica, and flavorant (for example, Flavor Peppermint) are sifted in vibratory sifter. Next, at step 404, the mixture is blended in a Conta blender and a flavored blend is obtained at step 406. In some instances, 50% of the flavored blend is unloaded from the Conta blender.

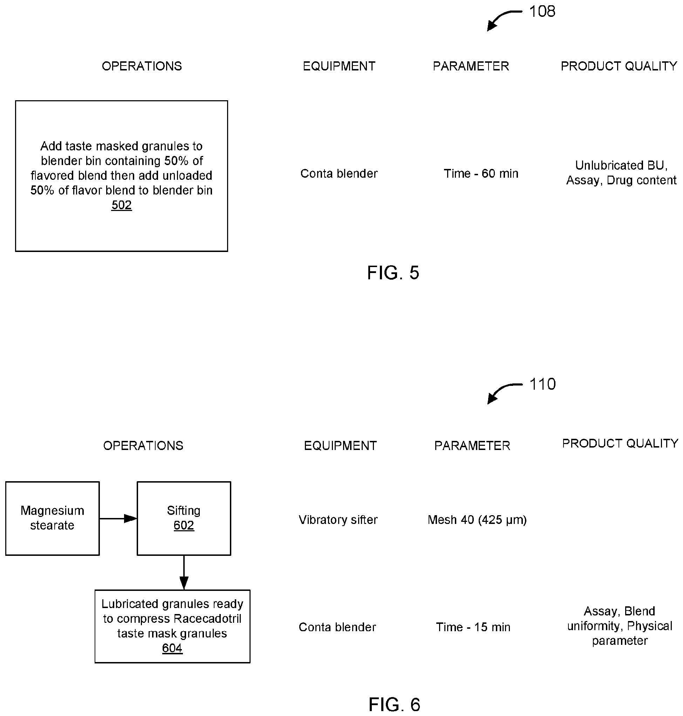

Now referring again to FIG. 1, at step 108, the process 100 comprises preparing an intermediate blend of taste masked granules. The step 108 is described in detail in FIG. 5. At step 502, the taste masked Racecadotril granules are added to the remaining 50% of flavored blend followed by addition of unloaded 50% of flavored blend in the Conta blender to form an intermediate blend of taste masked Racecadotril granules.

Now referring again to FIG. 1, at step 110, the process 100 comprises lubricating the intermediate blend of taste masked Racecadotril granules. The step 110 is described in detail in FIG. 6. At step 602, magnesium stearate is sifted in vibratory sifter. Next, at step 604, the intermediate blend of taste masked Racecadotril granules are lubricated in the Conta blender to form lubricated taste masked Racecadotril granules.

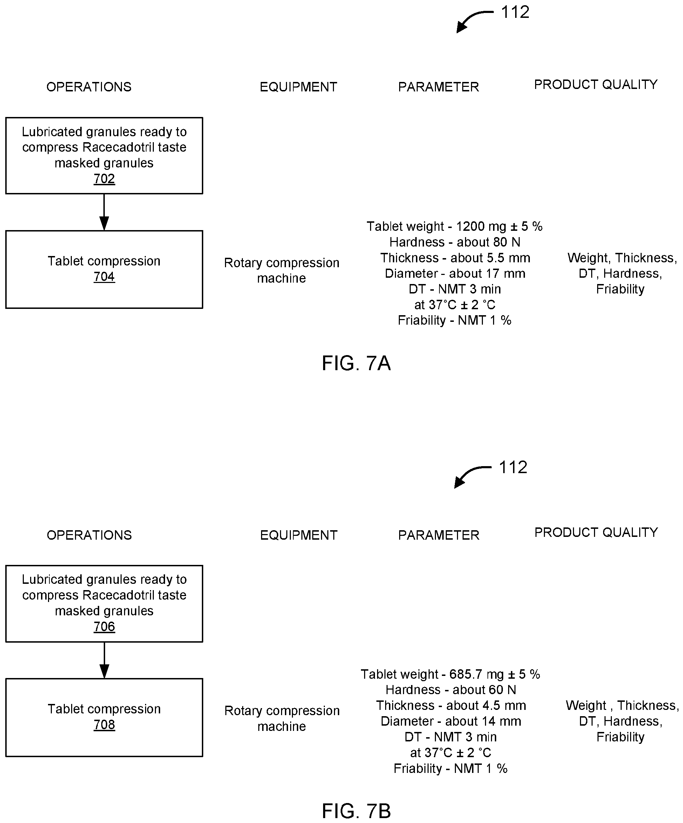

Now referring again to FIG. 1, at step 112, the process 100 comprises compressing the lubricated taste masked Racecadotril granules to form taste masked Racecadotril tablet of 175 mg and 100 mg. The step 112 is described in detail in FIG. 7A and FIG. 7B for preparation of tablets of strength 175 mg and 100 mg respectively. Referring to FIG. 7A, at step 702, the lubricated taste masked Racecadotril granules are ready for compression. At step 704, the lubricated taste masked Racecadotril granules are passed to a rotary compression machine at pre-determined parameters to form a tablet of 175 mg. As used herein, in a non-limiting example, pre-determined parameters include tablet weight = 1200 mg ± 5 %, hardness = about 80 Newton, thickness =about 5.5 mm, diameter = about 17 mm, disintegration time = NMT 3 min, at temperature 37°C ± 2 °C, and friability = NMT 1 %. Although particular pre-determined parameters are mentioned above, it will be evident to a person skilled in the art to modify predetermined parameters based on obvious variations.

Referring to FIG. 7B, at step 706, the lubricated taste masked Racecadotril granules are ready for compression. At step 708, lubricated taste masked Racecadotril granules are passed to a rotary compression machine at pre-determined parameters to form a tablet of 100 mg. As used herein, in a non-limiting example, pre-determined parameters include tablet weight = 685.7 mg ± 5 %, hardness = about 60 Newton, thickness = about 4.5 mm, diameter = about 14 mm, disintegration time = NMT 3 min, at temperature 37°C ± 2 °C, and friability = NMT 1 %. Although particular pre-determined parameters are mentioned above, it will be evident to a person skilled in the art to modify predetermined parameters based on obvious variations.

Conventional instruments like blenders, sifters, coating instruments, granulators and the compression machines are used for the manufacturing of taste masked tablet compositions.

The granulation operation may be performed using apparatus such as, for example, a fluidized-bed granulator, an agitation granulator, a biaxial granulator, or the like. Generally, the granulated mixture is preferably dried after granulation in any pharmaceutical acceptable manner. Drying of the granulate for example can be performed in one of the following ways: trays, fluid bed, and microwave assist/vacuum/gas stripping (one pot processing). The granulation may be carried out by suitable aqueous or non-aqueous solvents. Coating is applied to the granules in a fluid bed coater.

In one embodiment, the Racecadotril tablet composition is in the form of an orally disintegrating tablet (ODT) that can be consumed orally.

In another embodiment, the Racecadotril tablet composition is in the form of a dispersible tablet (DT) that can be dissolved in liquid to prepare extemporaneous suspension to be consumed.

The description of the present invention of pharmaceutical composition and process for preparation thereof is further illustrated by the following non-limiting example. A person skilled in the art would recognize that, the specific example is intended to illustrate, not limit, the scope of the present invention.

### EXAMPLE 1

In Example 1, a process for preparing taste masked Racecadotril tablet is described. Particularly, the process is described for preparing tablets having a strength of 175 mg per tablet and 100 mg per tablet.

The process is initiated by preparing Racecadotril granules by mixing and wet granulation. First, the binder solution is prepared by adding 0.38g of polyvinylpyrrolidone (for example, PVPK-30) in purified water and stirring for 10 minutes to get a clear solution. Thereafter, a process of sifting is carried out in a mixture of 14.58g of Racecadotril, 10.94g of mannitol (for example, Mannitol Fine 35) and 1.28g of anhydrous colloidal silica (for example, Aerosil 200) and sifted in a Vibratory Sifter. Thereafter, the binder solution is obtained.

Thereafter, the granulation is carried out in a rapid mixer granulator by mixing the binder solution with the mixture of received after the sifting process. Drying is carried out in a fluidized bed coater. As used herein, in a non-limiting example, pre-determined parameters include an inlet temperature of about 35°C to about 40°C and product temperature of about 25°C to about 30°C. 0.82g of anhydrous colloidal silica (for example, Aerosil 200) is added to dried drug granules and is sifted to prepare the Racecadotril granules.

In TABLE 1A below, the different ingredients for preparing Racecadotril granules are depicted.

**TABLE 1A**

| Ingredients | Solution | Qty. (mg) for 175 mg per tablet | Qty. (mg) for 100 mg per tablet |
|---|---|---|---|
| Racecadotril | Active | 175.00 | 100.0 |
| Mannitol Fine 35 | Diluent | 131.31 | 75.03 |
| Aerosil 200 | Antistatic agent | 15.31 | 8.75 |
| PVPK -30 | Binder | 4.60 | 2.63 |
| Purified water* | Solvent | Q.S | Q.S |

The next step in the process comprises applying taste masked coating suspension is applied on the Racecadotril granules to obtain taste masked Racecadotril granules. 0.875g of polyethylene glycol (for example, PEG 6000) is added in warm purified water and dissolved using stirring process to get polyethylene glycol solution. Next, 4.38g of amino alkyl methacrylate copolymer (for example, Eudragit E 100) is added into isopropyl alcohol and stirred to get a copolymer solution. Both the above solution (polyethylene glycol solution and copolymer solution) are mixed by continuous stirring process in a pneumatic stirrer to get clear solution. Further, 1.75g of anhydrous colloidal silica (Aerosil 200) is added to the above solution to obtain a taste masking coating suspension. Next, the Racecadotril granules are processed in a fluidized bed coater.

Thereafter, the prepared coating suspension is applied on Racecadotril granules in fluidized bed coater at pre-determined parameters to form coated granules. As used herein, in a non-limiting example, pre-determined parameters include an inlet temperature of about 35°C to about 40°C, product temperature of about 25°C to about 30°C, fluidization of air volume of about 250-500 CFM, spraying rate of the prepared coating suspension of about 100 to about 300 g/min and atmospheric pressure of about 0.5 to about 2.0 kg/cm² to get smooth uniform granules. Although particular pre-determined parameters are mentioned above, it will be evident to a person skilled in the art to modify predetermined parameters based on obvious variations.

Thereafter, the coated granules are dried in the fluidized bed coater at the pre-determined parameters. As used herein, in a non-limiting example, pre-determined parameters include an inlet temperature of about 30°C to about 35°C, product temperature of about 25°C to about 30°C, fluidization of air volume of about 250-500 CFM. Although particular pre-determined parameters are mentioned above, it will be evident to a person skilled in the art to modify predetermined parameters based on obvious variations. Finally, the coated granules are sifted in a vibratory sifter to obtain taste masked Racecadotril granules.

In TABLE 1B, the different ingredients for applying taste mask coating suspension on the Racecadotril granules to obtain taste masked Racecadotril granules are depicted.

**TABLE 1B**

| Ingredients | Solution | Qty. (mg) for 175 mg per tablet | Qty. (mg) for 100 mg per tablet |
|---|---|---|---|
| Aerosil 200 | Antistatic Agent | 9.78 | 5.59 |
| Eudragit E 100 | Film Forming agent | 52.50 | 30.00 |
| PEG 6000 | Pore former | 10.50 | 6.00 |
| Aerosil 200 | Antistatic Agent | 21.00 | 12.00 |
| Isopropyl alcohol* | Solvent | Q.S | Q.S |
| Purified water* | Solvent | Q.S | Q.S |

Next, the process comprises preparing a flavored blend. Mixture of 46.5g of mannitol (for example, mannitol granular such as PEARLITOL^{®} SD 200), 7.0g of microcrystalline cellulose (for example, MCC 102 (Vivapur pH102)), 5.0g of croscarmellose sodium (for example, VIVASOL^{®} GF), 2.5g of sweetener (for example, sucralose), 1.5g of sweetener (for example, aspartame), 0.5g of anhydrous colloidal silica (for example, Aerosil 200), and 1.0g of flavorant (for example, flavor Peppermint) are sifted in vibratory sifter. Further, the mixture is blended in a Conta blender and a flavored blend is obtained. In some instances, 50% of the flavored blend is unloaded from the Conta blender.

Next, the process comprises preparing an intermediate blend of taste masked granules. The taste masked Racecadotril granules are added to the remaining 50% of flavored blend followed by addition of unloaded 50% of flavored blend in the Conta blender to form an intermediate blend of taste masked Racecadotril granules.

In TABLE 1C below, the different ingredients for preparing flavored blend and blending the taste masked Racecadotril granules with the flavored blend to obtain an intermediate blend of taste masked Racecadotril granules are depicted.

**TABLE 1C**

| Ingredients | Solution | Qty. (mg) for 175 mg per tablet | Qty. (mg) for 100 mg per tablet |
|---|---|---|---|
| Mannitol granular (PEARLITOL^{®} SD 200) | Diluent | 558.00 | 318.85 |
| MCC (Vivapur pH102) | Cushioning agent | 84.00 | 48.00 |
| Croscarmellose sodium (VIVASOL^{®} GF) | Disintegrating Agent | 60.00 | 34.29 |
| Sucralose | Sweetener | 30.00 | 17.14 |
| Aspartame | Sweetener | 18.00 | 10.29 |
| Aerosil 200 | Antistatic agent | 6.00 | 3.43 |
| Flavor Peppermint | Flavor | 12.00 | 6.86 |

Next, the process comprises lubricating the intermediate blend of taste masked Racecadotril granules. 1.0g of magnesium stearate is sifted in vibratory sifter. Thereafter, the intermediate blend of taste masked Racecadotril granules are lubricated in the Conta blender to form lubricated taste masked Racecadotril granules.

In TABLE 1D below, the different ingredients for lubricating the intermediate blend of taste masked Racecadotril granules with magnesium stearate to form lubricated taste masked Racecadotril granules are depicted.

**TABLE 1D**

| Ingredient | Solution | Qty. (mg) for 175 mg per tablet | Qty. (mg) for 100 mg per tablet |
|---|---|---|---|
| Magnesium stearate | Lubricant | 12.00 | 6.86 |

Next, the process comprises compressing the lubricated taste masked Racecadotril granules to form taste masked Racecadotril tablet of 175 mg and 100 mg. The process initiates by passing the lubricated taste masked Racecadotril granules to a rotary compression machine to form a tablet of 175 mg and 100 mg. As used herein, in a non-limiting example, different pre-determined parameters are used for the tablet of 175 mg. For the tablet of 175 mg, tablet weight = 1200 mg ± 5 %, hardness = about 80 Newton, thickness =about 5.5 mm, diameter = about 17 mm, disintegration time = NMT 3 min, at temperature 37°C ± 2 °C, and friability = NMT 1 %. Although particular pre-determined parameters are mentioned above, it will be evident to a person skilled in the art to modify predetermined parameters based on obvious variations. As used herein, in a non-limiting example, different pre-determined parameters are used for the tablet of 100 mg. For the tablet of 100 mg, tablet weight = 685.7 mg ± 5 %, hardness = about 60 Newton, thickness = about 4.5 mm, diameter = about 14 mm, disintegration time = NMT 3 min, at temperature 37°C ± 2 °C, and friability = NMT 1 %. Although particular pre-determined parameters are mentioned above, it will be evident to a person skilled in the art to modify predetermined parameters based on obvious variations.

The following table provides a percentage by weight of the composition of the taste masked Racecadotril tablet (175 mg and 100 mg).

**TABLE 2**

| Ingredients | Relative Qty. |
|---|---|
| | % w/w |
| Racecadotril | 14.58 |
| Mannitol Fine 35 | 10.94 |
| Mannitol granular (PEARLITOL^{®} SD 200) | 46.5 |
| Aerosil 200 | 4.35 |
| PVPK -30 | 0.38 |
| Eudragit E 100 | 4.38 |
| Purified Water* | Q.S |
| PEG 6000 | 0.875 |
| Isopropyl alcohol* | Q.S |
| Purified Water* | Q.S |
| MCC (Vivapur pH102) | 7.0 |
| Croscarmellose sodium (VIVASOL^{®} GF) | 5.0 |
| Sucralose | 2.5 |
| Aspartame | 1.5 |
| Flavor Peppermint | 1.0 |
| Magnesium Stearate | 1.0 |
| Total | 100.0 |

The granules were compressed to form a tablet using suitable parameters

**TABLE 3: Evaluation of Tablet parameters**

| SN | Parameters | 100 mg | 175 mg |
|---|---|---|---|
| 1 | Average weight of tablet | 685.7 mg ± 5 % | 1200 mg ± 5 % |
| 2 | Thickness | 4.5 mm | 5.5mm |
| 3 | Hardness | 60 Newton | 80 Newton |
| 4 | Disintegration time | Not more than 3 Minutes at 37 ± 2°C | Not more than 3 Minutes at 37 ± 2°C |
| 5 | Friability | Not more than 1.0 % | Not more than 1.0 % |

The foregoing descriptions of specific embodiments of the present disclosure have been presented for purposes of illustration and description. They are not intended to be exhaustive or to limit the present disclosure to the precise forms disclosed, and obviously many modifications and variations are possible in light of the above teaching. The embodiments were chosen and described in order to best explain the principles of the present disclosure and its practical application, to thereby enable others skilled in the art to best utilize the present disclosure and various embodiments with various modifications as are suited to the particular use contemplated. (It is understood that various omission and substitutions of equivalents are contemplated as circumstance may suggest or render expedient, but such are intended to cover the application or implementation without departing from the spirit or scope of the claims of the present disclosure).

## Claims

1. A taste masked Racecadotril tablet composition comprising:
13 to 15 % w/w of Racecadotril;
0.3 to 0.4 % w/w of polyvinylpyrrolidone;
3 to 5 % w/w of anhydrous colloidal silica;
50 to 60 % w/w of mannitol;
0.5 to 1 % w/w of polyethylene glycol;
4 to 5 % w/w amino alkyl methacrylate copolymer;
5 to 10 % w/w of microcrystalline cellulose; and
3 to 7 % w/w of croscarmellose sodium;
wherein the % w/w is with respect to the total weight of the composition.

2. The taste masked Racecadotril tablet composition according to Claim 1, **characterized in that**
polyvinylpyrrolidone is PVPK-30.

3. The taste masked Racecadotril tablet composition according to Claim 1 or 2,
**characterized in that**
amino alkyl methacrylate copolymer is Eudragit E 100.

4. The taste masked Racecadotril tablet composition according to one of the previous Claims,
**characterized in that**
the composition further comprises a sweetener and a flavorant.

5. The taste masked Racecadotril tablet composition according to Claim 4, **characterized in that**
the sweetener is at least one of sucralose and aspartame.

6. The taste masked Racecadotril tablet composition according to Claim 4 or 5,
**characterized in that**
the flavorant is Flavor peppermint.

7. The taste masked Racecadotril tablet composition according to one of the previous Claims,
**characterized in that**
the composition further comprises a lubricant, wherein the lubricant is magnesium stearate.

8. The taste masked Racecadotril tablet composition according to one of the previous Claims,
**characterized in that**
the amount of Racecadotril is 100 mg to 200 mg per tablet.

9. The taste masked Racecadotril tablet composition according to one of the previous Claims,
**characterized in that**
the tablet is an orally disintegrating tablet.

10. The taste masked Racecadotril tablet composition according to one of Claims 1 to 8,
**characterized in that**
the tablet is a dispersible tablet.

11. The taste masked Racecadotril tablet composition according to Claim 9, **characterized in that**
the disintegration time for the tablet is less than 60 secs.

12. The taste masked Racecadotril tablet composition according to one of the previous Claims,
**characterized in that**
the hardness of the tablet ranges from 60-80 Newton.

13. A process for preparing taste masked Racecadotril tablet, the process comprising the steps of:
- preparing Racecadotril granules by mixing and wet granulation of racecadotril, mannitol, anhydrous colloidal silica and polyvinylpyrrolidone;
- applying a taste masking coating suspension consisting of an amino alkyl methacrylate copolymer, polyethylene glycol and anhydrous colloidal silica on the Racecadotril granules to obtain taste masked Racecadotril granules;
- preparing a flavored blend by mixing mannitol, microcrystalline cellulose, croscarmellose sodium, sweetener, anhydrous colloidal silica and flavorant;
- blending the taste masked Racecadotril granules with the flavored blend to obtain an intermediate blend of taste masked Racecadotril granules;
- lubricating the intermediate blend of taste masked Racecadotril granules with magnesium stearate to form lubricated taste masked Racecadotril granules; and
- compressing the lubricated taste masked granules to form taste masked Racecadotril tablet.

14. The process according to Claim 13,
**characterized in that**
the tablet is an orally disintegrating tablet or a dispersible tablet.

15. The process according to Claim 13 or 14,
**characterized in that**
taste masked Racecadotril granules has a particle size distribution between 100 µm to 500 µm.
